# EUROPÄISCHE PATENTSCHRIFT

(11) **EP 1 200 126 B1**
(45) Veröffentlichungstag und Bekanntmachung des Hinweises auf die Patenterteilung: **22.04.2009**
(21) Anmeldenummer: 00956124.2
(22) Anmeldetag: 27.07.2000
(51) Int. Cl.: A61K 39/395, A61K 31/70, A61K 31/47, A61P 31/18, C07K 16/28

(54) **VERWENDUNG CD28 SPEZIFISCHER MONOKLONALER ANTIKÖRPER ZUR HERSTELLUNG EINER PHARMAZEUTISCHEN ZUSAMMENSETZUNG ZUR BEHANDLUNG VON VIRUSINFEKTIONEN**
USE OF CD28 SPECIFIC MONOCLONAL ANTIBODIES FOR PRODUCING A PHARMACEUTICAL COMPOSITION FOR TREATING VIRUS INFECTIONS
UTILISATION D'ANTICORPS MONOCLONAUX SPECIFIQUES DE CD28 POUR PREPARER UNE COMPOSITION PHARMACEUTIQUE APPROPRIEE AU TRAITEMENT D'INFECTIONS VIRALES

(30) Priorität: 13.08.1999 DE 19939653
(43) Veröffentlichungstag der Anmeldung: 02.05.2002
(73) Patentinhaber: Archem Service Company Limited, Gibraltar (GI)
(72) Erfinder: HÜNIG, Thomas, D-97078 Würzburg (DE)
(74) Vertreter: Jungblut, Bernhard Jakob
(86) Internationale Anmeldenummer: PCT/DE2000/002596
(87) Internationale Veröffentlichungsnummer: WO 2001/012224

(56) Entgegenhaltungen:
- WO-A-94/06473
- WO-A-98/17313
- DE-A- 19 722 888
- DATABASE AIDSLINE [Online] AN=1998:8037, 1998 M. HEZAREH ET AL.: "Differential T cell induction of productive HIV replication in samples from patients on prolonged suppressive therapy." XP002150584 & CONFERENCE ON RETROVIRUSES AND OPPORTUNISTIC INFECTIONS, Bd. 5, 1998, Seite 183
- U. HENGGE ET AL.: "Randomized, controlled phase II trial of subcutaneous interleukin-2 in combination with highly active antiretroviral therapy (HAART) in HIV patients." AIDS, Bd. 12, Nr. 17, 1998, Seiten F225-F234, XP000877329 in der Anmeldung erwähnt
- T-W. CHUN ET AL.: "Effect of interleukin-2 on the pool of latently infected, resting CD4+ T cells in HIV-1-infected patients receiving highly active anti-retroviral therapy." NATURE MEDICINE, Bd. 5, Nr. 6, Juni 1999 (1999-06), Seiten 651-655, XP002150581 New York, NY, VSA in der Anmeldung erwähnt
- T-W. CHUN ET AL.: "Quantification of latent tissue reservoirs and total body viral load in HIV-1 infection." NATURE, Bd. 387, Nr. 6629, 8. Mai 1997 (1997-05-08), Seiten 183-188, XP002150582 London, GB in der Anmeldung erwähnt
- E. BARKER ET AL.: "CD28 costimulation increases CD8+ cell suppression of HIV replication." THE JOURNAL OF IMMUNOLOGY, Bd. 159, Nr. 10, 15. November 1997 (1997-11-15), Seiten 5123-5131, XP002150583 Baltimore, MD, VSA
- SUNTHARALINGAM ET AL: "Cytokine Storm in a Phase 1 Trial of the Anti-CD28 Monoclonal Antibody TGN1412" NEW ENGLAND JOURNAL OF MEDICINE, Bd. 355, Nr. 10, 7. September 2006 (2006-09-07), Seiten 1018-1028,

## Beschreibung

### Gebiet der Erfindung

Die Erfindung betrifft eine pharmazeutischen Zusammensetzung in der Ausführungsform als Präparat oder Präparatepaket zur Behandlung von Virusinfektionen beim Menschen, bei welchen T-Lymphozyten infiziert sind.

### Hintergrund der Erfindung

Das HIV durchläuft einen Lebenszyklus, in welchem es in verschiedenen Latenzstadien vorliegen kann. Ein erstes Latentzstadium wird als präintegrativ bezeichnet und meint, daß das HIV zwar in die Wirtszelle importiert und ggf. zumindest teilweise der reversen Transkription unterzogen, jedoch nicht in den Zellkern als Provirus eingebaut ist. Dieses präintegrative Latenzstadium kann latent funktional bleiben über eine Mehrzahl von Wochen bis zur Einbüßung der Funktionsfähigkeit. Die präintegrative Latenz erfordert, daß die Wirtszelle ruht. Ein weiteres Latenzstadium wird als postintegrativ bezeichnet und meint, daß das HIV als Provirus zwar in den Zellkern intergriert worden ist, die Wirtzelle jedoch beispielsweise aufgrund von Deaktivierung ruht und folglich keine Virusreplikation stattfindet. Die postintegrative Latenz ist vergleichsweise langzeitstabil und hält bis zu einer Aktivierung der Wirtszelle an. Eine Aktivierung von latentem (post- oder präintegrativ) HIV enthaltenden Wirtzellen erfolgt durch verschiedene Stimuli mit der Folge der Aktivierung auch der Virusreplikation, wobei die Wirtszelle zerstört und Virus in die Körperflüssigkeit freigesetzt wird. Die vorstehenden Erläuterungen gelten grundsätzlich für alle Retroviren. Virus in einem latenten Stadium wird folgend Latenzvirus genannt.

Bisherige Therapieansätze mit Reverse Transkriptase Inhibitoren und ggf. Protease Inhibitoren unterdrücken die Virusvermehrung nach Aktivierung des Latenzvirus. Somit verschwindet zwar freies HIV unter Therapie aus der Zirkulation, ruhende Leukozyten enthalten jedoch weiterhin Latenzvirus (T.W. Chun et al., Proc. Natl. Acad. Sci. USA, 94:13193-13197 (1997); D. Finzi et al., Science, 278:1295-1300 (1997); J.K. Wong et al., Science, 278:1291-1295 (1997)). Die Folge ist ein Wiedererscheinen replikationsfähiger Viren bei Unterbrechung der Therapie (M.D. de Jong et al., AIDS, 11:F79-84 (1997)) und Fortschreiten der Erkrankung. Die Therapie mit Reverse Transkriptase Inhibitoren und ggf. Protease Inhibitoren ist jedoch toxisch und eine insofern notwendige lebenslange Behandlung hat daher ihre Grenzen bzw. Bedenken. Zudem ist die Behandlung mit Reverse Transkriptase Inhibitoren und ggf. Protease Inhibitoren mit beachtlichen Kosten verbunden.

### Stand der Technik

Als Konsequenz aus der vorstehenden grundsätzlichen Problematik wurde vorgeschlagen, latentes HIV der Therapie mit Reverse Transkriptase Inhibitoren und Protease Inhibitoren durch gleichzeitige Behandlung mit immunstimulierenden Agentien, die das latente HIV aktivieren, zugänglich zu machen ("flush out"; O.J. Cohen et al., J. Am. Med. Assoc., 280:87-88 (1998); J. Cohen, Science, 279:1854-1855 (1998); D.D. Ho, Science, 280:1866-1867 (1998); L.K. Schrager et al., J. Am. Med. Assoc., 280:67-71 (1998)). Konkret zeigt die Literaturstelle T.W. Chun et al., Nature Medicine, Volume 5, Number 6, pp. 651-655 (1999), daß durch Einsatz des T-Zellwachstumsfaktors Interleukin 2 (IL-2) zusammen mit der HAART Therapie (siehe hierzu unten) eine signifikante Reduktion der Menge an replikationskompetentem HIV in den ruhenden T-Lymphozyten gefunden wird. Allerdings exprimiert der größte Teil ruhender CD4 T-Lymphozyten keine Rezeptoren für den Wachstumsfaktor IL-2. Diese Zellen und damit das darin enthaltene latente HIV können deshalb durch die Darreichung von IL-2 nicht erreicht werden. Die grundsätzliche vorstehende Problematik bleibt daher bestehen und ist allenfalls geringfügig abgemildert. Hinzu kommt, daß die Darreichung von IL-2 mit beachtlich störenden Nebenwirkungen verbunden ist, welche den Erfolg dieser Strategie noch weiter relativieren.

Die verbreitetste Therapie unter Verwendung von Reverse Transkriptase Inhibitoren ist die HAART Therapie ("hochaktive anti-retrovirale Therapie"). Diese besteht in der kombinierten Anwendung von zwei Reverse Transkriptase Inhibitoren, z.B. die Nukleosidanaloge AZT (bzw. Zidovudine) und 3TC (bzw. Lamivudine), zusammen mit einem oder mehreren Protease Inhibitoren. Ein Beispiel für einen Protease Inhibitor ist IDV (Indinavir). Bezüglich der HAART Therapie, der darin verwendeten Substanzen und des Behandlungsplans wird auf die folgenden Literaturstellen verwiesen: J. Laurence, HAART Regiments: Do the effects last? in The AIDS Reader 7(6):84-85 (1997); R.M. Gulick et al., N. Engl. J. Med., 337:734-739 (1997); S.M. Hammer et al., N. Engl. J. Med., 337:725-733 (1997); und F.J. Jr. Palella et al., N. Engl. J. Med., 338:853-860 (1998). Weitere Beispiele für geeignete Reverse Transkriptase Inhibitoren sind die Nukleosidanaloge d4T. (Stavudine), ddl (Didanosine) und ddC (Zalcitabine) sowie die nicht-Nukleosidanaloge DLV (Delavirdine) und NVP (Nevirapine). Weitere Beispiele für geeignete Protease Inhibitoren sind NFV (Nelfinavir), RTV (Ritonavir) und SQV (Saquinavir).

Aus der Literaturstelle WO 98/54225 sind humanverträgliche monoklonale Antikörper, welche für Human-CD28 spezifisch sind und Human-T-Lymphozyten mehrer bis aller Untergruppen ohne Besetzung eines Antigenrezeptors der Human-T-Lymphozyten und somit antigenunspezifisch aktivieren, bekannt. Bezüglich weiterer Hintergrundinformation wird auf die in dieser Literaturstelle genannten Zitate verwiesen. Aus dieser Literaturstelle ist es auch bekannt, diese monoklonalen Antikörper zur Behandlung von Erkrankungen mit pathologisch erniedrigten CD4-T-Zellzahlen, wie beispielsweise AIDS, zu verwenden. Hintergrund dieser Verwendung ist, daß mittels dieser Antikörper die CD4-T-Zellzahlen wieder angehoben werden können. Eine Verbindung mit der Darreichung von Reverse Tranksriptase Inhibitoren und ggf. Protease Inhibitoren ist nicht gezogen.

Aus der Literaturstelle Database AIDSLINE, AN = 1998:8037, 11 Hezareh et al., ist es bekannt, dass klassische AntiCD3/CD28 T-Zell Stimulierung zu einer Erhöhung der HIV-Virusproduktion führt.

### Aufgabe der Erfindung

Gegenüber dem nächstliegenden Stand der Technik gemäß T.W. Chun et al., Nature Medicine, Volume 5, Number 6, pp. 651-655, liegt der Erfindung das technische Problem zugrunde, eine pharmazeutische Zusammensetzung bzw. einen Behandlungsplan zu entwickeln, womit einerseits zumindest der größte Teil der HIV Latenzviren, wenn nicht alle, aktiviert (und damit durch Virus Inhibitoren hemmbar und letzlich zerstörbar gemacht) wird und andererseits die Nebenwirkungen reduziert werden.

### Grundzüge der Erfindung

Die Erfindung lehrt den fegenstand des Anspruchs 1. Es versteht sich, daß die Wirkstoffkomponenten in pharmazeutisch wirksamen Dosen eingesetzt werden.

Die pharmazeutische zusammensetzung kann optional einen von b) verschiedenen Reverse Transkriptase Inhibitor und optional einen von c) verschiedenen Protease Inhibitor enthalten. Neben den vorstehenden Wirkstoffkomponenten können noch weitere Wirkstoffe und/oder für die galenische Herrichtung zweckmäßige oder notwendige Stoffe enthalten sein.

Die Erfindung ist geeignet zur Behandlung von Virusinfektionen mit Lentiviren, insbesondere AIDS, bei Menschen.

Die Erfindung beruht zunächst auf der Erkenntnis, daß durch die Aktivierung eines Großteils der T-Lymphozyten auch ruhendes (Latenz-) Virus aktiviert und so durch Reverse Transkriptase Inhibitoren und Protease inhibitoren zerstörbar gemacht wird. Hieran anschließend beruht die Erfindung auf der weiteren überraschenden Erkenntnis, daß die parallele Anwendung von (toxischen) Reverse Transkriptase Inhibitoren, beispielsweise der (toxischen) HAART Therapie, die Aktivierung der T-Lymphozyten nicht konterkariert. Dies hat im Ergebnis eine doppelte Bedeutung und folglich synergistische Wirkung. Einerseits ist so die Aktivierung des Großteils der T-Lymphozyten trotz paralleler HAART Therapie mit der Folge der Vernichtung praktisch des gesamten Latenzvirus-Reservoirs durch die HAART Therapie gewährleistet. Andererseits wird gleichzeitig die ohnehin pathologisch erniedrigte Anzahl der T-Lymphozyten wieder angehoben mit der Folge einer Stabilisierung des Immunsystems. Hinzu kommt noch, daß mit den erfindungsgemäßen Mitteln vermutlich auch nicht-T-Zell Reservoire für Latenzviren (z.B. Makrophagen) indirekt durch die starke generelle Stimulierung des Immunsystems bzw. der T-Zellen mit der entsprechenden Zytokinfreisetzung aktiviert werden und so auch diese Latenzviren aktiviert und zerstört werden können; eine weitere Synergie.

Letztendlich wird erreicht, daß nicht nur das freie Virus praktisch vollständig ausgeschaltet wird, sondern auch praktisch das ganze Latenzvirus Reservoir durch Aktivierung der Zerstörung zugänglich gemacht wird. Dadurch braucht die HAART Therapie nicht mehr lebenslang, zumindest jedoch nur noch in sehr großen Zeitabständen, eingesetzt zu werden. Ein weiterer überraschender Vorteil gegenüber dem nächstliegenden Stand der Technik ist, daß die erfindungsgemäß eingesetzten Antikörper nach ersten Untersuchungen in Tiermodellen keine Nebenwirkungen zu erzeugen scheinen. Insgesamt wird eine wesentlich effektivere Ausschaltung des Virus in Verbindung mit einer beachtlich verbesserten Befindlichkeit des Patienten bereits während der Therapie erreicht.

In diesen Zusammenhängen mag angemerkt werden, daß mit den erfindungsgemäß eingesetzten monoklonalen Antikörpern in der Tat sämtliche CD4 T-Zellen zur Proliferation angeregt werden konnen. Nur eine Subpopulation von CD8 T-Lymphozyten, die CD28 nicht exprimieren, kann nicht durch aktivierende CD28-spezifische Reagenzien aktiviert werden. Allerdings stellt diese auch kein wesentliches Reservoir von HIV dar, da der primäre Rezeptor für HIV das CD4 Molekül ist.

### Definitionen

Als monoklonale Antikörper sind Antikörper bezeichnet, die von Hybrid-Zellinien (sog. Hybridomen) produziert werden, die durch Fusion einer Antikörpern produzierenden B-Zelle tierischer oder menschlicher Herkunft mit einer geeigneten Myelom Tumorzelle entstanden sind. Im Rahmen dieser Beschreibung sind mit dem Ausdruck der monoklonalen Antikörper auch deren Derivate umfaßt.

Als CD28 wird ein auf T-Lymphozyten menschlicher und tierischer Herkunft exprimiertes Zelloberflächenmolekül bekannter Aminosäuresequenz bezeichnet, dem im Rahmen der internationalen "Human Leukocyte Typing Workshops" das Kürzel CD28 gegeben wurde.

Mit Aktivierung von T-Lymphozyten ist die Vermehrung der Stoffwechselaktivität, Vergrößerung des Zellvolumens, Synthese immunologisch wichtiger Moleküle und Eintritt in die Zellteilung (Proliferation) von T-Lymphozyten auf einen äußeren Reiz hin gemeint. Beispielsweise werden diese Vorgänge durch Besetzung des CD28-Moleküls auf T-Zellen durch besondere CD28-spezifische monoklonale Antikörper ausgelöst. Die Aktivierung von T-Lymphozyten mit den beschriebenen Begleiterscheinungen ist Teil der physiologischen Immunreaktion, kann dort aber in pathologischen Situationen außer Kontrolle geraten (lymphoproliferative Erkrankungen), oder unzureichend sein (Immundefizienz).

Konstante Komponenten eines Antikörpers sind Bereiche, die nicht für die Antigenerkennung bedeutsam sind, im Gegensatz zu den variablen Bereichen, die die Antigenspezifität eines Antikörpers definieren. Konstante Komponenten unterscheiden sich jedoch bei Antikörpern verschiedener Arten und folglich auch Tieren und Menschen. Die konstanten Bereiche eines Antikörpers müssen jenen von Antikörpern eines Organismus entsprechen, der mit den Antikörpern behandelt werden soll, um verträglich zu sein.

Unter Derivaten von monoklonalen Antikörpern sind Modifikationen des monoklonalen Antikörpers zu verstehen, die durch übliche biochemische oder gentechnische Manipulationen erzeugt wurden. Dies ist beispielsweise gegeben mit der Humanisierung eines monoklonale Antikörpers der Maus durch partiellen Ersatz struktureller (konstanter) Komponenten des Maus-Antikörpers durch solche eines menschlichen. Derivate sind weiterhin monoklonale Antikörper, welche zwar chemisch verändert sind, jedoch dennoch die im Rahmen der Erfindung erläuterten Funktionen ausüben. Gemeinsames Kriterium ist stets die CD28 Spezifität mit stimulatorischem Effekt.

Analoge sind Substanzen, die keine monoklonalen Antikörper sind, jedoch die im Rahmen der Erfindung erläuterten Funktionen ausüben. Beispiele hierfür sind "geschneiderte" hochspezifische synthetische Proteine oder RNA bzw. DNA Moleküle (z.B. Aptamere, insbesondere gegen Nukleinsäurespaltende Enzyme stabilisierte Aptamere bzw. RNA oder DNA Moleküle). Gemeinsames Kriterium ist stets die CD28-Spezifität mit stimulatorischem Effekt.

Unter einer Determinante ist der Bereich eines Moleküls zu verstehen, der durch die Bindungsspezifität eines oder mehrerer Antikörper definiert wird.

Der Ausdruck der therapeutisch aktiven Dosis bezeichnet im Zusammenhang mit Virus Inhibitoren, beispielsweise Reverse Transkriptase Inhibitoren (und ggf. Protease Inhibitoren), eine Dosis, die zu einer signifikanten Verringerung der Menge an replikationsfähigem Virus ab einem definierten Zeitraum nach Darreichung der Virus Inhibitoren an einen Patienten oder in einem Testsystem führt, verglichen mit der Menge an replikationsfähigem Virus nach gleichem Zeitraum und gleicher Anfangsmenge an replikationsfähigem Virus, jedoch ohne irgendeine Darreichung.

Der Ausdruck der therapeutisch aktiven Dosis bezeichnet im Zusammenhang mit erfindungsgemäß eingesetzten Antikörpern eine Dosis, die zu einer signifikanten Erhöhung der CD4 T-Zellzahlen und/oder der Expression von serologisch nachweisbaren Aktivierungsmarkern (CD25, CD45R0, CD71) nach einem definierten Zeitraum in einem Organismus oder Testsystem, welchem die Antikörper dargereicht wurden, verglichen mit respektiven Werten nach gleichem Zeitraum und gleicher Anfangswerte, jedoch ohne irgendeine Darreichung.

Humanverträglich bezeichnet Antikörper, welche humanisiert sind. Es mag hier angemerkt werden, daß nicht humanisierte und folglich nicht unter die hier getroffene Definition der humanverträglichen Antikörper fallende Antikörper durchaus zur Therapie beim Menschen angewandt werden können. Bei der Therapie des Menschen können insofern alle Antikörper eingesetzt werden, welche über einen bestimmten Zeitraum keine unerwünschten Immunreaktionen auslösen, wie beispielsweise durch Bestimmung von anti-Immunglobulin Antikörper als Abbruchkriterium nachweisbar.

Als Virus Inhibitor ist jeder Wirkstoff bezeichnet, welcher in eine beliebige Stufe des Lebenszyklus eines Virus direkt oder indirekt hemmend eingreift. Hierfür kommen neben Reverse Transkriptase Inhibitoren und Protease Inhibitoren beispielsweise auch Inhibitoren der Zelloberflächenrezeptoren in Frage, an welche ein Virus andockt, oder Inhibitoren aller positiv regulatorischen Proteine bzw. Prozesse eines Virus, einschließlich Inhibitoren zellulärer positiv regulatorischer Substanzen, welche auf die long terminal repeats eines Virus wirken. Grundsätzlich kommen auch Wirkstoffe in Frage, welche keine Inhibitoren sind, sondern negativ regulatorische Proteine bzw. Prozesse eines Virus induzieren; solche Wirkstoffe sind von dem Ausdruck Virus Inhibitor ebenfalls umfaßt.

Der Begriff der kontinuierlichen Darreichung einer Wirkstoffkomponente b) und/oder c) und/oder d) und/oder e) meint, daß ein zu dieser Komponente anzuwendender (in sich ggf. diskontinuierlicher) Behandlungsunterplan kontinuierlich fortgeführt wird. Insofern schließt der Ausdruck der kontinuierlichen Darreichung beispielsweise bei der HAART Therapie auch ein Variation und individuelle Anpassung der Wirkstoffkomponenten bzw. deren Dosierung in Verfolg der kontinuierlichen Darreichung ein.

### Detaillierte Darstellung der Erfindung

Folgend werden zweckmäßige oder bevorzugte Ausführungsformen der Erfindung angegeben und näher erläutert.

Die Erfindung ist geeignet zur Behandlung von Erkrankungen, wobei CD4 T-Lymphozyten, insbesondere Human-CD4 T-Lymphozyten, infiziert sind, wobei die monoklonalen Antikörper für Human-CD28 spezifisch sind und wobei die monoklonalen Antikörper optional humanverträglich sind. Bei der Behandlung von Infektionen von Human-CD4-Lymphozyten, also des Menschen, müssen die Antikörper Human-CD28 spezifisch sein, nicht jedoch notwendigerweise humanverträglich. Die Erfindung ist beispielsweise anwendbar, wenn die Virusinfektion eine Infektion mit Retrovirus, insbesondere Lentivirus, beispielsweise HIV, ist.

Zweckmäßig ist es, wenn der Reverse Trankriptase Inhibitor ein Pyrimidin-Nukleosidanalog, vorzugsweise 3'-Azido-3'-desoxythymidin (AZT oder Zidovudin), ist und optional weiterhin zusätzlich andere hiervon verschiedene Nukleosidanaloge, vorzugsweise 3TC, in der pharmazeutischen Zusammensetzung enthalten sind. Die pharmazeutische Zusammensetzung enthält zusätzlich einen Proteaseinhibitor und optional weiterhin zusätzlich andere, hiervon verschiedene Proteaseinhibitoren'. Mit einer Wirkstoffkombination aus zumindest zwei Reverse. Transkriptase Inhibitoren und zumindest einem Protease Inhibitor arbeitet die HAART Therapie.

Erfindungsgemäß eingesetzte monoklonale Antikörper sind auf die verschiedensten Weisen erhältlich. Eine verwendbare Ausführungsform gemäß der Ausführungsbeispiele ist erhältlich sind durch A) Herstellung von zur Produktion von monoklonalen Human-CD28 spezifischen Tier-Antikörpern befähigten Hybridomzellen im Wege einer Immunisierung mit nicht-T-Tumorzelllinien, auf welchen Human-CD28 exprimiert ist, B) ggf. Humanisierung der aus Hybridomzellen gemäß Stufe A erhältlichen monoklonalen Tier-Antikörper durch biochemischen oder gentechnologischen Austausch konstanter Komponenten der Tierantikörper gegen analoge konstante Komponenten eines menschlichen Antikörpers bzw. Austausch den Komponeneten entsprechender Gene der Hybridomzellen, C) Sezernierung der monoklonalen Antikörper in Hybridomzell-Kulturen und Isolierung der monoklonalen Antikörper daraus oder Produktion der monoklonalen Antikörper durch Injektion der Hybridomzellen in Tiere, beispielsweise Mäuse, und Isolierung der monoklonalen Antikörper aus der Körperflüssigkeit der Tiere. Die zur Produktion von monoklonalen Human-CD28 spezifischen Tier-Antikörpern befähigten Hybridomzellen sind erhältlich durch a) Schaffung eines Plasmids mittels Insertion von Human-CD28 cDNA in den pHβAPr-1-neo Vector nach Excision des SalI-HindIII Fragments und Herstellung von Protoplasten aus Escherichia coli (MC1061), welche das Plasmid tragen, b) Fusionierung der Protoplasten mit Maus A20J und/oder L929 Tumorzellen mittels Polyethylenglykol, c) Kultivierung der in Stufe b erhaltenen transfektierten Zellen, d) Screenen der transfektierten Maus A20J und/oder L929 Zellen auf die Expression von Human-CD28 und Selektion von Human-CD28 exprimierenden Maus A20J und/oder L929 Zellen, e) Immunisierung von BALB/c Mäusen mit den Human-CD28 exprimierenden Maus A20J und/oder L929 Zellen, f) Entnahme von Milzzellen der so immunisierten Mäuse und Fusionierung der Milzzellen mit Zellen der Zellinie X63-Ag 8.653 mittels Polyethylenglykol, g) Selektierung der so erhaltenen Hybridomzellen mit der Maßgabe, daß im Überstand selektierter Hybridomzellen Antikörper enthalten sind, die an Human-CD28 exprimierende Maus A20J und/oder L929 Zellen binden und h) Kultivierung/Subclonierung der in Stufe g erhaltenen selektierten Hybridomzellen. Anstelle der Stufen a) bis d) können selbstverständlich aber auch andere dem Fachmann geläufige Expressionsysteme eingesetzt werden. Human-CD28 cDNA ist frei erhältlich von Dr. A. Aruffo und Dr. B. Seed, die die Sequenz und auch folgende Literaturstelle veröffentlicht haben: Aruffo, A., and Seed, B, 1987, "Molecular cloning of a CD28 cDNA by a high efficiency COS cell expression system", Proc. Natl. Acad. Sci. USA, 84:8573. Dieser Literaturstelle ist daher im einzelnen die Herstellung der Human-CD28 cDNA entnehmbar. Darüberhinaus kann unschwer jeder Fachmann mit Hilfe der in der Genbank deponierten Sequenz und der Polymerasekettenreaktion sehr einfach und schnell einen Human-CD28 cDNA Klon herstellen. Der pHßAPr-1-neo Vector ist frei erhältlich von den Authoren der Literaturstelle Gunning, P, et al., 1987, "A human βactin expression vector system directs high-level accumulation of antisense transcripts", Proc. Natl. Acad. Sci. USA, 84:4831. "neo" steht dabei für Neomycin-Resistenz. Die Stufe c) wird daher in Anwesenheit von Neomycin durchgeführt. Die vorstehend angesprochenen Zellinien und/oder Mikroorganismen sind frei verfügbar und käuflich erwerbbar bei der American Type Culture Collection (ATCC). Bezüglich Escherichia coli (MC1061) wird ergänzend auf die Literaturstelle Meissner, P.S., et al., 1987, "Bacteriophage gamma cloning system for the construction of directional cDNA libraries", Proc. Natl. Acad. Sci. USA, 84:4171, verwiesen.

Die grundsätzliche Vorgehensweise bei der Herstellung von Hybridomzellen, bei der Humanisierung sowie bei der Produktion der monoklonalen Antikörper aus (humanisierten) Hybridomzellen ist dem Fachmann gut vertraut und braucht hier nicht näher erläutert zu werden. Grundsätzlich sind alle insbesondere für die Herstellung der Hybridomzellen üblichen, bekannten und frei verfügbaren Zellinien einsetzbar. Zur Herstellung der monoklonalen Antikörper kommt grundsätzlich neben der folgend beschriebenen Vorgehensweise die dem Fachmann im Detail gut geläufige rekombinante Expression in Frage.

Einsetzbare monoklonale Antikörper sind aber auch auf anderen Wegen zugänglich. So kann beispielsweise die Immunisierung in Stufe A) gegen lösliches bzw. gelöstes rekombinantes Human-CD28 erfolgen. Die Humanisierung kann.entbehrlich gemacht werden, indem bei der Herstellung der Hybridomzellen Tiere eingesetzt werden, die gentechnisch so verändert sind, daß die gebildeten Antikörper bereits die humanen konstanten Komponenten aufweisen. Ein völlig anderer Ansatz zur Herstellung monoklonaler Antikörper besteht darin, daß die Antigen-Bindungsdomänen (z.B. menschlicher) Antikörper in einer hochkomplexen Bakteriophagen-Bibliothek gentechnisch exprimiert werden, aus welcher geeignete Bindungsdomänen durch ihre Affinität für CD28 isoliert und zu vollständigen Antikörpern ergänzt werden können.

Hinsichtlich der pharmazeutischen Zusammensetzung können die Wirkstoffkomponenten b) und c) entsprechend der HAART Therapie ausgewählt, dosiert und darreichungsfertig hergerichtet werden. Grundsätzlich sind alle bestehenden und zukünftig entwickelten Varianten der HAART Therapie oder einer anderen Therapie brauchbar, solange nicht durch eine oder mehrere Wirkstoffkomponenten die aktivierende Wirkung der monoklonalen Antikörper (über-) kompensiert wird. Im Rahmen-derzeitiger Therapieansätze ist oft erfolgversprechend, wenn..:die Wirkstoffkomponente b) ein Pyrimidin-Nukleosidanalog, vorzugsweise 3'-Azido-3'-desoxythymidin, ist und/oder die 3TC umfasst. Es kann zweckmäßig sein, wenn die Wirkstoffkomponenten b) und c) Bestandteil einer ersten Paketkomponente und die Wirkstoffkomponente a) Bestandteil einer zweiten Paketkomponente sind.

Bei der Anwendung der Erfindung in einem Behandlungsverfahren können die Wirkstoffkomponenten b) und c) entsprechend der HAART Therapie ausgewählt, dosiert und dargereicht werden, wobei die Wirkstoffkomponente a) vor, zusammen oder nach den Wirkstoffkomponenten b) und c) dargereicht werden. Im einzelenen können die Wirkstoffkomponenten b) und c) köntinuierlich und die Wirkstoffkomponente a) einmal oder mehrmals in zeitlichen Abständen mit Ruhepausen, dargereicht werden. Die Wirkstoffkomponente b) kann ein Pyrimidin-Nukleosidanalog, vorzugsweise 3'-Azido-3'-desoxythymidin, und/oder 3TC sein.

Im Rahmen eines Behandlungsplans kann die Darreichung der Wirkstoffkomponente b) im Rahmen eines Behandlungsunterplans erfolgen, welcher die HAART Therapie ist. Zunächst erfolgt die HAART Grundtherapie über eine Dauer von 1 bis 12 Monate, vorzugsweise 2 bis 6 Monate, höchstvörzugsweise 2 bis 4 Monate, beispielsweise 3 Monate (1 Monat = 30 Tage). Während dieser Zeit können CD4 T-Zellzahlen und/oder Virusbelastung und/oder Latenzvirus (beispielsweise gemäß der Literaturstelle T.W. Chun et al., Nature, 387:183-188 (1997))regelmäßig überprüft werden und kann auf Basis dieser Ergebnisse die HAART Therapie individuell auf den Patienten angepaßt werden (durch Wahl bzw. Austausch und Kombination der Reverse Transkriptase Inhibitoren und/oder der Protease Inhibitoren sowie der jeweiligen Dosierungen). In einem ersten Zyklus kann dann eine vorzugsweise i.v. Injektion von Antikörpern in einer Dosierung von 0,1 bis 50, vorzugsweise 0,5 bis 20, höchstvorzugsweise 0,5 bis 5 mg/kg Körpergewicht erfolgen unter Aufrechterhaltung der HAART Therapie. Die vorstehende Dosis kann auf einmal oder in 2 bis 10, vorzugsweise 2 bis 5, Teilmengen über einen Zeitraum von 1h bis 1 Monat, vorzugsweise 1 Tag bis 5 Tage, gleichmäßig oder ungleichmäßig verteilt verabreicht werden. In einer hieran anschließenden Ruhepause (unter Aufrechterhaltung von HAART) von 1 Tag bis 6 Monaten, vorzugsweise 0,5 bis 2 Monaten, beispielsweise 1 Monat, kann eine Überwachung der vorstehend zur Grundtherapie genannten Werte und/oder des Blutbildes und/oder der Blutwerte und/oder klinisch internistischer Befunde und/oder der Bildung von anti-Immunglobulin Antikörpern (anti-Tier Ig bei nicht humanisierten Antikörpern, anti-idiotypisch nach Humanisierung) erfolgen. Nach Ablauf der Ruhepause kann bei Bedarf der Zyklus beginnend mit der Darreichung des Antikörpers wiederholt werden. Wenn PBMC (Lymphozyten plus Monozyten) virus- und latenzvirus- bzw. provirusfrei sind, kann bei Einverständnis des Patienten eine Lymphknotenbiopsie zur Verifizierung durchgeführt werden. Bei positivem Ergebnis (positiv = Detektion von Virus bzw. Latenzvirus bzw. Provirus) können weitere Zyklen der vorstehend beschriebenen Art angeschlossen werden. Bei negativem Ergebnis kann die Darreichung von HAART Wirkstoffen und von Antikörpern abgesetzt werden. Es empfiehlt sich, nach Absetzung weiter Kontrolluntersuchungen der vorstehenden Art in bestimmten zeitlichen Abständen durchzuführen, um ggf. die Behandlung wieder aufzunehmen. Die vorstehenden Ausführungen gelten entsprechend für ein Behandlungsverfahren.

Eingesetzt werden können im Rahmen der Erfindung beispielsweise der monoklonale Antikörper CMY-2, erhältlich aus Hybridomzellen gemäß Hinterlegung DSM ACC2353, oder der käuflich von der Firma ALEXIS Deutschland GmbH, D-35305 Grünberg, erhältliche und von der Firma Ancell Corporation, USA, hergestellte Klon ANC28.1/5D10 oder eine vorzugsweise humanisierte bzw. humanverträgliche Variante des Klons ANC28.1/5D10.

Erfindungsgemäß eingesetzte monoklonale Antikörper können insbesondere Spezifität für Determinanten des menschlichen CD28-Moleküls aufweisen, die auf dem natürlicherweise exprimierten CD28-Molekül schwer zugänglich sind und deren Besetzung durch die monoklonale Antikörper zur Aktivierung der T-Zellen führt.

Die galenische Herrichtung von Wirkstoffkomponenten oder von Mischungen daraus für die verschiedenen Verabreichungsformen ist dem Fachmann gut bekannt und braucht hier nicht näher erläutert zu werden.

Im Rahmen der Erfindung können optional noch zusätzliche Wirkstoffe präsent sein oder verwendet werden, welche von den vorstehend genannten und gemäß der Grundkonzeption der Erfindung eingesetzten Wirkstoffen verschieden sind. Solche zusätzliche Wirkstoffe sind beispielsweise Wirkstoffe, welche eventuellen Nebenwirkungen entgegenwirken. Lediglich beispielhaft seien anti-TNF-Antikörper (TNF = Tumor Nekrose Faktor) im Falle einer proinflammatorischen TNF Reaktion genannt. Zusätzliche Wirkstoffe sind weiterhin Wirkstoffe, welche im Zusammenhang mit der Expansion des Immunsystems hilfreich sein können. So kann beispielsweise durch Gabe von IL-2 die Proliferation von CD8-Zellen induziert bzw. verstärkt werden. Generell können als zusätzliche Wirkstoffe immunmodulierende Wirkstoffe eingesetzt werden nach Maßgabe des immunologischen (Detail- bzw. Neben-) Prozesses welcher im Zusammenhang mit der Erfindung zweckmäßigerweise gefördert (oder gehemmt) wird. Beispiel hierfür sind Oligonukleotide enthaltend CpG Motive (D. Klinman et al., Proc. Natl. Acad. Sci. USA, 93:2879-2883 (1996)).

Im folgenden wird die Erfindung anhand von Ausführungsbeispielen näher erläutert. U.a. wird die Herstellung erfindungsgemäß eingesetzter monoklonaler Antikörper beschrieben. In diesen Ausführungsbeispielen werden auch Screeningverfahren im einzelnen deutlich, mit welchen erfindungsgemäße monoklonale Antikörper bzw. zugrundeliegende Hybridomzellen selektiert werden können. Aus den folgenden Beispielen werden auch die therapeutischen Wirkungen deutlich.

### Beispiel 1: Herstellung und Wirkung eines ersten erfindungsgemäß verwendbaren monoklonalen Antikörpers.

### 1.1 Allgemeine Informationen.

Die dargestellten Experimente bzw. die Beispiele zu den Wirkungen von "direkten" CD28-spezifischen monoklonalen Antikörpern wurden im Tiermodell der Ratte durchgeführt, wobei als Beispiel für einen "klassischen" CD28-spezifischen Antikörper der monoklonale Antikörper JJ319 und als Beispiel für einen "direkt" aktivierenden der monoklonale Antikörper JJ316 eingesetzt wird. Beide Antikörper sind frei verfügbar und käuflich erwerbar von der Firma Pharmingen, San Diego, USA. JJ319 und JJ316 Antikörper sind im übrigen erhältlich gemäß der Literaturstelle M. Tacke et al., immunology, 1995, 154: 5121-5127, auf welche hiermit ausdrücklich Bezug genommen wird, auch im Hinblick auf Details der Herstellung von Hybridomzellen und monoklonalen Antikörpern.

### 1.2: Herstellung monoklonaler Antikörper

In diesem Beispiel wird die Herstellung erfindungsgemäßer, d.h. human-CD28-spezifischer monoklonaler Antikörper näher erläutert. Diese werden folgend auch als CMY-2 bezeichnet. Human CD28 aus einer cDNA Bibliothek wurde in A20J und/oder L929 Zellinien rekombinant exprimiert. Zunächst wurde hierzu ein Plasmid mittels Insertion von Human-CD28 cDNA in den pHβAPr-1-neo Vector nach Excision des SalI-HindIII Fragments geschaffen. Aus Escherichia coli (MC1061) wurden Protoplasten hergestellt, welche das Plasmid tragen. Dann erfolgte eine Fusionierung der Protoplasten mit Maus A20J und/oder L929 Tumorzellen mittels Polyethylenglykol. Die so erhaltenen transfektierten Zellen wurden auf übliche Weise kultiviert. Anschließend erfolgte ein Screenen der transfektierten Maus A20J und/oder L929 Zellen auf die Expression von Human-CD28 und Selektion von Human-CD28 exprimierenden Maus A20J und/oder L929 Zellen.

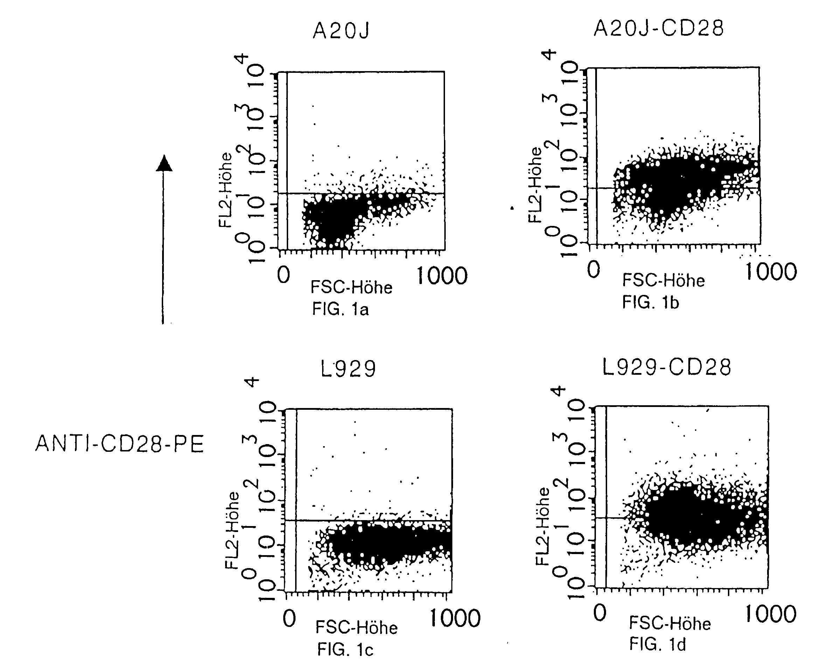

Der Nachweis der erfolgreichen Expression erfolgte mit Hilfe eines konventionellen, kommerziell erhältlichen fluoreszenzmarkierten Antikörpers mit Spezifität für Human CD28 (9.3-Phykoerythrin). Als Negativkontrolle wurden nicht transfizierte A2OJ- bzw. L929-Zellen mit dem gleichen Antikörper gefärbt. Die Transfektanten (A20J-CD28 und L929-CD28) zeigten eine höhere Fluoreszenzintensität. Da nicht alle Zellen CD28 positiv waren, wurden CD28-positive Zellen subkloniert und zur Immunisierung verwendet. Wie in Fig. 1 an der Verschiebung der Punktwolken nach oben in den beiden rechten Diagrammen erkennbar, reagierten diese Zellen mit dem käuflichen Antikörper, drückten also Human CD28 an ihrer Oberfläche aus.

Die A20J Human-CD28 Zellinie wurde zur Immunisierung von BALB/c Mäusen verwendet. Zellfusion und screening wurden wie folgt durchgeführt: i) Immunisierung von BALB/c Mäusen mit den Human-CD28 exprimierenden Maus A20J Zellen (Injektionen 6 x i.p. und anschließend 1 x i.v.). ii) Entnahme von Milzzellen der so immunisierten Mäuse und Fusionierung der Milzzellen mit Zellen der Zellinie X63-Ag 8.653 mittels Polyethylenglykol. iii) Selektierung der so erhaltenen Hybridomzellen mit der Maßgabe, daß im Überstand selektierter Hybridomzellen Antikörper enthalten sind, die an Human-CD28 exprimierende Maus A20J und/oder L929 Zellen binden.

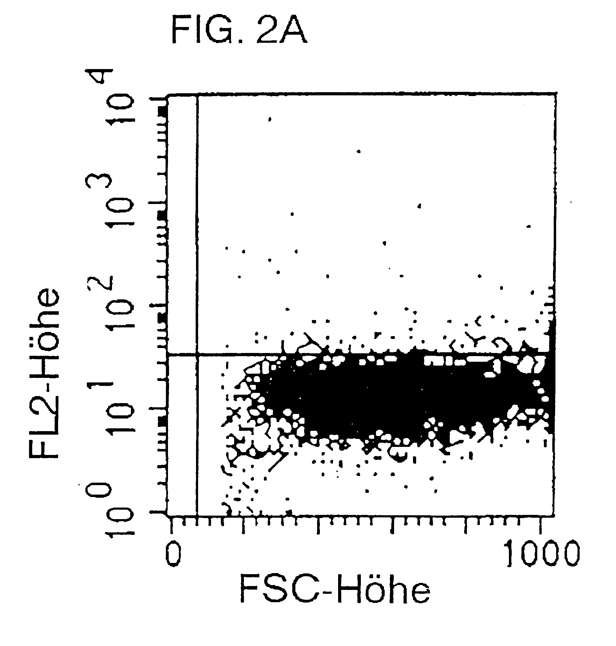

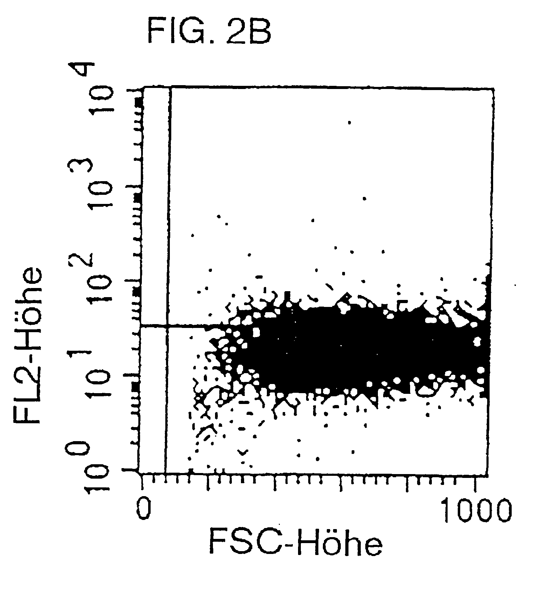

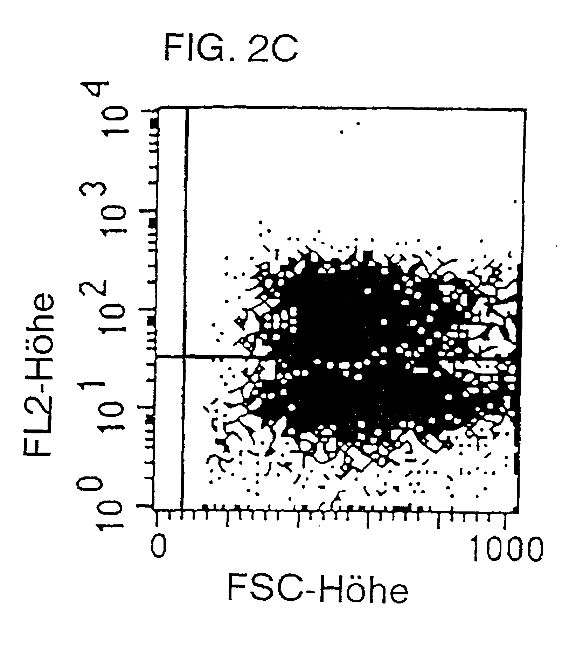

Als read-out diente die Anfärbung einer Mischung aus CD28 transfizierten und untransfizierten Maus L929 Tumorzellen. Fig. 2 zeigt, daß der auf diesem Weg isolierte monoklonale Antikörper CMY-2 transfizierte und untransfizierte Zellen durch unterschiedliche Fluoreszenzintensität unter- scheidet. Das differentielle Screening auf Antikörper gegen Human-CD28 erfolgte wie folgt. Je 50 µl Überstand von kultivierten Zellhybridomen wurden entnommen und mit einem Gemisch aus L929-Zellen und L929-CD28-Transfektanten 15 min inkubiert. Nach dem Waschen wurden die Zellen mit DaMIg-PE angefärbt. Teil A zeigt die Negativkontrolle. Die Zellen wurden nur mit DaMIg-PE inkubiert. Teil B zeigt die Färbung mit einem Überstand, der leicht positiv war, aber keinen Unterschied bei beiden Zellen zeigt. Teil C zeigt die mit einem Überstand von CMY-2 gefärbten Zellen.

In nicht dargestellten Experimenten wurden periphere Blutzellen des Menschen mit dem neu isolierten CMY-2 und dem "klassischen" CD28-spezifischen Antikörper 9.3 gefärbt. Es wurde ein identisches Expressionsmuster auf den Subpopulationen menschlicher Blutzellen gefunden.

Zusammengefaßt zeigen die Experimente, daß CMY-2 ein human CD28-spezifischer Antikörper ist.

CMY-2 wurde sodann mit aus peripherem Blut auf circa 80 % angereicherten menschlichen T-Lymphozyten auf klassische kostimulierende und auf "direkt" stimulierende Aktivität getestet. Die T-Zellproliferation wurde durch Einbau von ³H-Thymidin zwischen dem 2. und 3. Tag der Kultur gemessen. Folgende Ergebnisse wurden erzielt:

### Kostimulation:

| | |
|---|---|
| Unstimulierte Zellen | 276 cpm |
| CD3-spezifischer Antikörper | 3111 cpm |
| CD3-spezifischer Antikörper + CMY-2 | 51676 cpm |

### Direkte Stimulation:

| | |
|---|---|
| Solid-phase anti-mouse Ig | 379 cpm |
| Solid-phase anti-mouse Ig + Kontroll-mAk | 258 cpm |
| Solid-phase anti-mouse Ig plus CMY-2 | 19115 cpm |

Zur Erläuterung: Anti-CD3 sorgt für T-Zellrezeptor-Stimulation (CD3 ist Teil des TCR-Komplexes). CMY-2 wurde in Form eines nicht aufgereinigten Kulturüberstandes (50% Endvolumen) verwendet. Erfahrungsgemäß ist die dabei zu erwartende effektive mAk-Konzentration suboptimal für eine direkte Aktivierung, aber ausreichend für die Kostimulation. Das Experiment zeigt, daß CMY-2 direkt aktivierende Eigenschaften hat.

Hybridomzellen, welche CMY-2 produzieren, sind bei der DSMZ, Deutsche Sammlung von Mikroorganismen und Zellkulturen GmbH, Mascheroder Weg 1b, D-38124 Braunschweig, unter der Nummer DSM ACC2353 (20.05.1998) hinterlegt worden.

### 1.3 Proliferative Antwort auf den Antikörper aus 1.2.

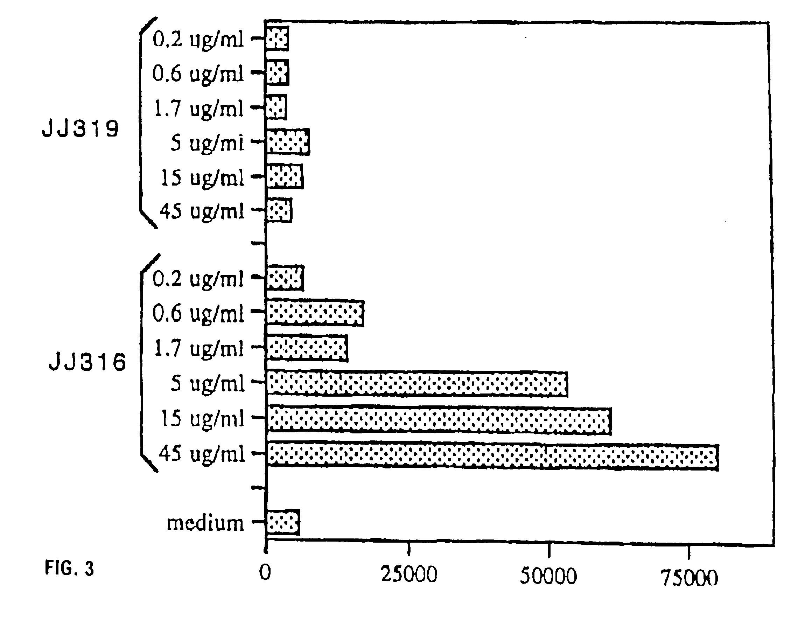

Fig. 3 zeigt die proliferative Antwort ungetrennter Lymphknotenzellen der Ratte auf den "direkt" stimulierenden CD28-spezifischen monoklonalen Antikörper (JJ316) und das Ausbleiben einer solchen Antwort bei Einsatz eines "klassischen " CD28-spezifischen monoklonalen Antikörpers (JJ319). Die Zellen wurden zwei Tage lang in 0,2 ml Medium (RPMI 1640, erhältlich von GIBCO/BRL, enthaltend 5 % FCS [fetal calf serum])in An- oder Abwesenheit der angegebenen Zusätze bei einer Dichte von 1 Mio. Zellen pro ml im begasten Brutschrank kultiviert. Die Zellteilungsaktivität wurde durch den Einbau radioaktiv markierten Thymidins (1 µCi/Ansatz für 16 Std., 1Ci = 37GBq, Bestimmung mit ß-Detektor) bestimmt.

Im Gegensatz zu veröffentlichten Resultaten (Siefken et al., Cellular Immunology, 1997, 176: 59-65) zeigt dieses Ergebnis, daß es für die T-Zell-Aktivierung durch direkt aktivierende CD28-spezifische monoklonale Antikörper nicht notwendig ist, diese artifiziell durch einen zweiten Antikörper miteinander zu vernetzen. Vielmehr reicht die Anwesenheit von nicht-T-Zellen aus lymphoiden Organen, nämlich von B-Lymphozyten und sogenannten akzessorischen Zellen, um eine direkte Aktivierung durch löslich zugegebene CD28-spezifische monoklonale Antikörper zu ermöglichen. Wahrscheinlich geschieht dies durch Bindung der monoklonale Antikörper an sogenannte Fc-Rezeptoren dieser nicht-T-Zellen. Dieses Ergebnis ist eine wichtige Voraussetzung für den therapeutischen Einsatz "direkt" stimulierender CD28-spezifischer monoklonale Antikörper, in dem eine artifizielle Vernetzung mit anti-Immunglobulin Antikörpern im Gesamtorganismus nicht praktikabel ist.

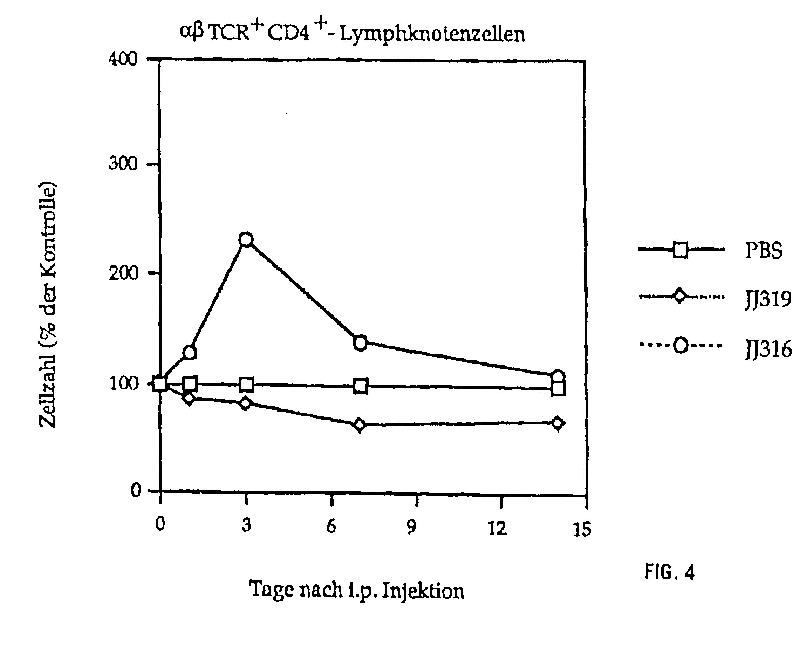

"Direkt" aktivierende CD28-spezifische monoklonale Antikörper führen zu einer Erhöhung der CD4 T-Zellzahl im intakten Organismus. Fig. 4 zeigt dies für Lymphknoten der Ratte, die am Tag 0 1 mg des "direkt" stimulierenden CD28-spezifischen monoklonale Antikörpers (JJ316) oder des "klassischen" CD28-spezifischen monoklonalen Antikörpers (JJ319) erhalten hatten. Mit erfindungsgemäßen direkt aktivierenden monoklonalen Antikörpern mit Spezifität für Human-CD28, und deren Fähigkeit, die Vermehrung von T-Lymphozyten zu stimulieren, werden ganz analoge Effekte erreicht. Im vorliegenden Beispiel ist die CD4 T-Zellzahl nur vorübergehend erhöht; das liegt daran, daß gesunde Tiere mit normalen CD4 T-Zellzahlen behandelt wurden. Die aufgrund der Proliferationsstimulierung "überschüssigen" Zellen werden durch homöostatische Mechanismen abgebaut.

Die vorstehend im einzelnen erläuterten Ergebnisse zeigen, daß eine Aktivierung der T-Lymphozyten erfolgt, und zwar ohne die Notwendigkeit weiterer Wirkstoffe.

### Beispiel 2: Verwendung erfindungsgemäß eingesetzter Antikörper in Verbindung mit der HAART Therapie

### 2.1: Allgemeine Informationen.

In den folgend beschriebenen Versuchen wurde anstelle des in Beispiel 1 beschriebenen monoklonalen Antikörpers der Antikörper Klon ANC28.1/5D10 der Firma Ancell Corporation, USA, vertrieben von der Firma ALEXIS Deutschland GmbH, Giessener Str. 12, D-35305 Grünberg, eingesetzt. Von diesen Antikörpern ist bekannt, daß sie in CD28 positiven Zellen IL-2 induzieren. Eine proliferationsinduzierende Aktivität dieses Antikörpers ist jedoch bislang nicht beschrieben worden. Dieser Antikörper wird folgend kurz als "aCD28" bezeichnet. In den folgenden Versuchen wird zu Vergleichszwecken ein monoklonaler Antikörper verwendet, welcher nicht "direkt" stimulierend ist, nämlich CD28.2.

Die Versuche wurde, sofern nicht anders angegeben, in humanen PBL oder in PBL von Rhesusaffen durchgeführt. Dieser Zellkulturansatz kommt der Situation im Gesamtorganismus nahe, weil der stimulierende Antikörper, wie für eine Therapie vorgesehen, in löslicher Form zugesetzt wird.

Bei allen Proliferationsexperimenten wurde gemessen durch einen Puls von 1µCi ³H-Tymidin für 16 Stunden zwischen Tag 3 und Tag 4 und Detektion mittels β-Detektor.

Sofern nicht anders angegeben wurden Zellen in 0,2 ml Medium (RPMI 1640, erhältlich von GIBCO/BRL, enthaltend 5% menschliches AB Serum) bei einer Dichte von 1 Mio. Zellen pro ml im begasten Brutschrank kultiviert.

### 2.2: Induktion von T-Zellproliferation in peripheren Blut-Lymphozyten und/oder Monozyten (PBMC) durch aCD28.

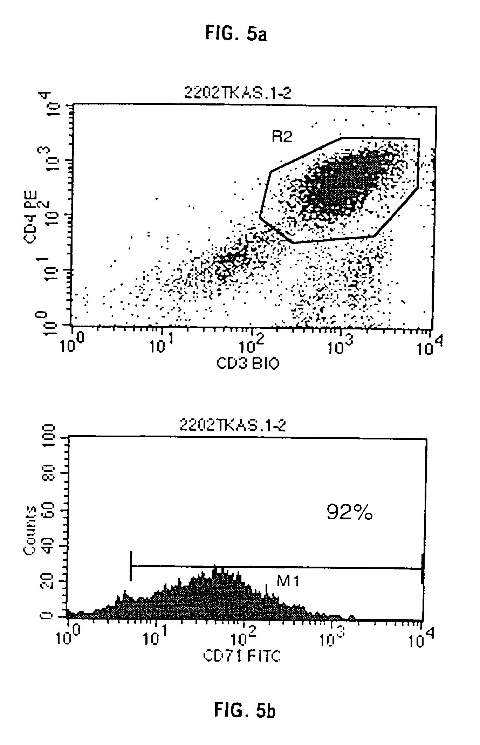

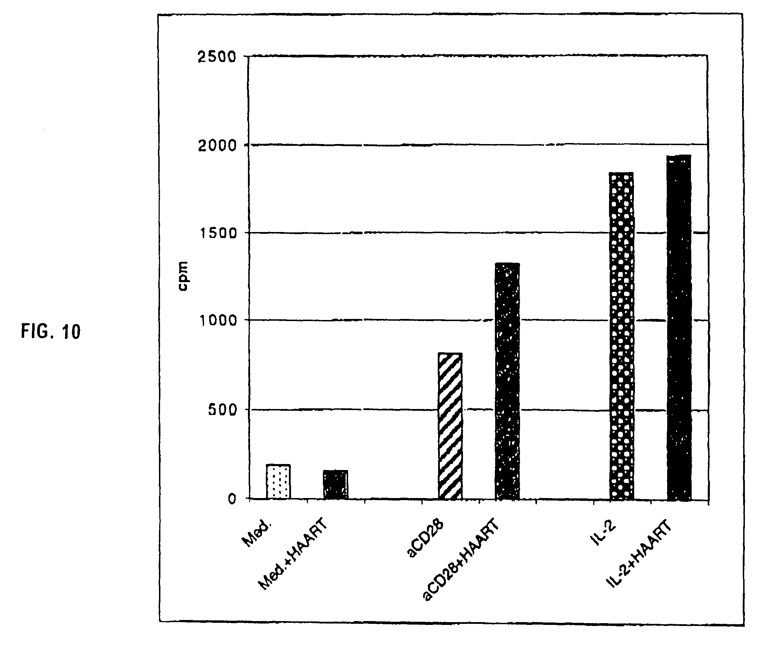

In der Fig. 5 sind Versuche zum Maß der Aktivierung der Proliferation von CD4 T-Zellen dargestellt. Der Nachweis erfolgte durch Immunfluoreszenz und Durchflußzytophotometrie (FACS) am Tage vier nach Stimulation der Zellen in vitro. Fig. 10a zeigt sogenannte "dotplots", wobei durch ein Gate (R2) die CD4 T-Zellen definiert werden (sie exprimieren CD3 und CD4). In der Fig. 10b ist in einem Histogramm die Anfärbung dieser CD4 T-Zellen mit einem monoklonalen Antikörper gegen den Transferinrezeptor (CD71) dargestellt. Die Expression dieses Oberflächenrezeptors charakterisiert proliferierende Zellen. Man erkennt, daß in einem Großteil, nämlich >90%, der CD4+ T-Lymphozyten die Proliferation durch aCD28 (direkt) induziert wird. Die Untersuchungen erfolgten mit 5µg/ml aCD28.

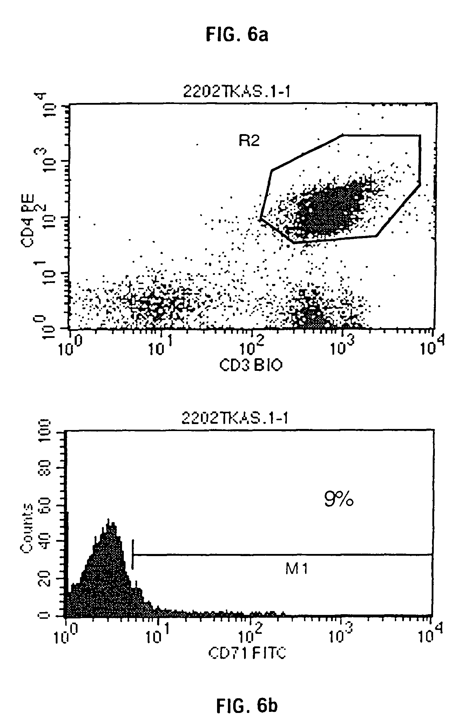

In der Fig. 6 sind entsprechende Darstellungen, jedoch ohne Stimulation, als Negativkontrollen gezeigt.

### 2.3: Vergleich der Stimulation der Proliferation von ungetrennten PBMC durch aCD28 und IL-2.

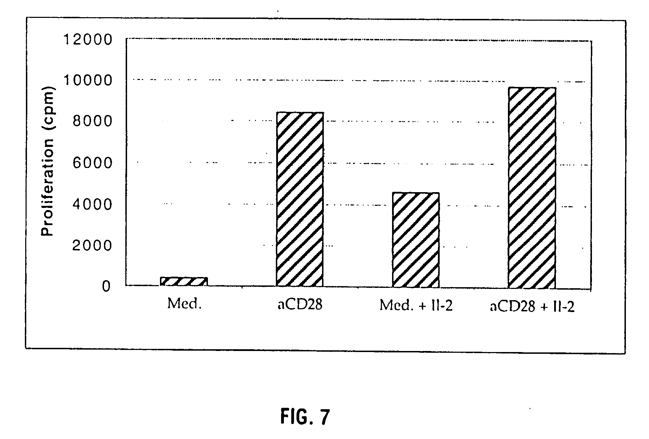

Die Figur 7 zeigt, daß die Proliferation ungetrennter PBMC von nicht infizierten menschlichen Spendern, gemessen am Einbau ³H-markierten Tymidins, durch aCD28 stärker ist als durch IL-2. Dies zeigt, daß mehr Zellen mittels aCD28 angesprochen werden als bei der in vivo Therapie mit HAART und IL-2 gemäß dem Stand der Technik. Die Kultivierung erfolgte in Gegenwart von 5µg/ml aCD28 bzw. 20 I.U./ml IL-2 in 96-well Rundboden Platten.

Wichtig ist darüber hinaus, daß IL-2 präferentiell CD8T-Lymphozyten zur Proliferation stimuliert, während durch aCD28 besonders CD4 T-Zellen angesprochen werden. Dies ist aus den Daten der Tabelle I ersichtlich: PBMC eines HIV infizierten Patienten wurden drei oder 10 Tage mit IL-2 bzw. aCD28 stimuliert. Durch IL-2, nicht aber durch aCD28 erfolgte eine deutliche Verschiebung zu Gunsten der CD8 T-Zellen.

**Tabelle I**

| | nach 3 | Tagen | nach 10 | Tagen |
|---|---|---|---|---|
| | %CD44 | %CD8 | %CD4 | %CD8 |
| Medium | 51 | 49 | 50 | 50 |
| IL-2 | 46 | 54 | 27 | 73 |
| aCD28 | 57 | 43 | 62 | 38 |

### 2.4: Stimulation der Proliferation von PBMC aus Virusinfizierten Organismen.

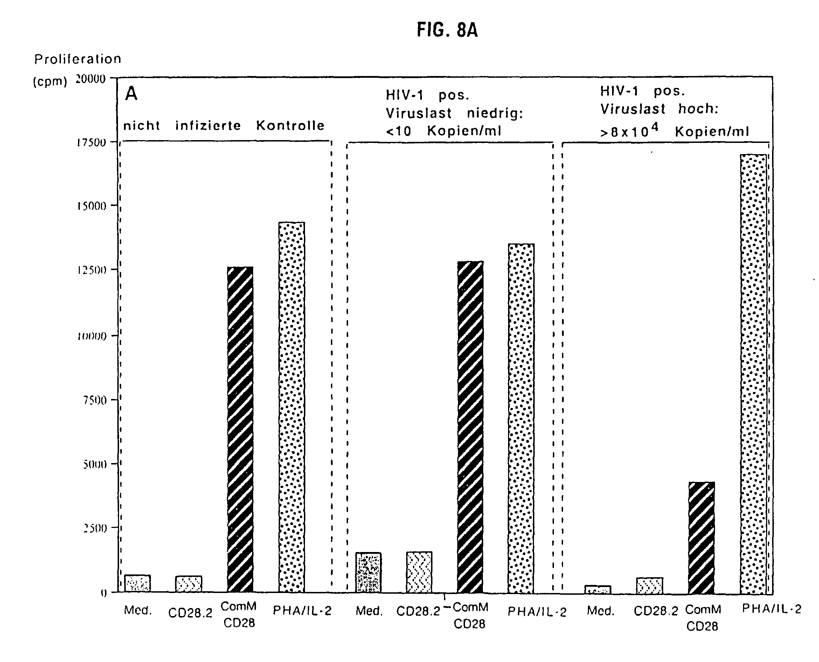

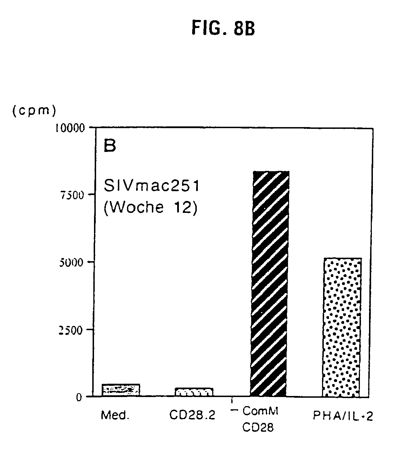

Figur 8A zeigt die aktivierende Wirkung von aCD28 auf die Proliferation von PBMC aus HIV-1 infizierten Personen, Figur 8B Entsprechendes für PBMC aus SIV-infizierten Rhesusaffen. Eingesetzt wurde eine Menge von 5µg/ml aCD28. In allen Fällen konnte starke Proliferation induziert werden, die bei massiver HIV-Infektion jedoch am geringsten ausfiel. Dies hängt vermutlich mit der damit einhergehenden massiven Induktion der Virusvermehrung und damit einhergehender T-Zellzerstörung zusammen, die bei erfindungsgemäßer Behandlung bei gemeinsamer Darreichung von HAART Wirkstoffen vermieden wird (siehe auch Beispiel 2.6a und Fig. 10).

### 2.5: Aktivierung der Proliferation in Gegenwart von HAART-Wirkstoffen.

PBLs einer HIV-1 infizierten Person wurden nicht, mit HAART Wirkstoffen (Zidovudine + Didanosine + Saquinavir; 0,1µM) allein, mit aCD28 (5µg/ml) allein oder mit beidem behandelt. Nach 6 Tagen wurde die Proliferation gemessen.

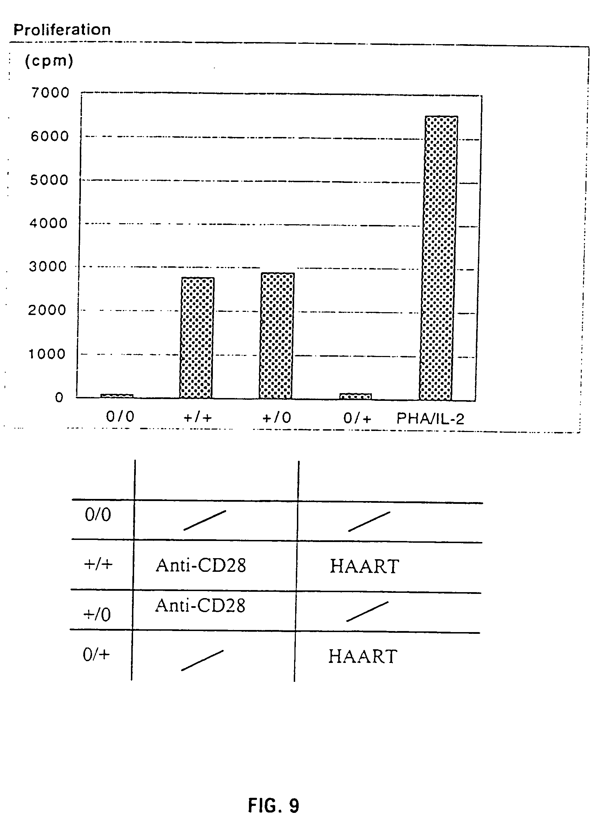

Mann erkennt in der Figur 9, in welcher die Ergebnisse dargestellt sind, daß die Induktion der Proliferation mittels aCD28 durch Anwesenheit von HAART nicht berührt ist.

### 2.5a Förderung der CD28-, nicht jedoch der IL-2-induzierten T-Zellproliferation.

PBMC eines HIV-1 infizierten Patienten wurden wie vorstehend beschrieben stimuliert. Die Proliferation wurde von Tag 3 zu Tag 4 gemessen. Wie bei infizierten Patienten bisweilen beobachtet, induziert IL-2 eine stärkere Proliferation als CD28, die jedoch vorwiegend die nicht HIV belasteten CD8 T-Zellen betrifft (siehe Tabelle I). Diese wird nicht durch HAPRT beeinflußt, während die aCD28-induzierte durch HAART verbessert wird. (siehe Fig. 10)

### 2.6: Einfluß von aCD28/HAART auf die Virusreplikation.

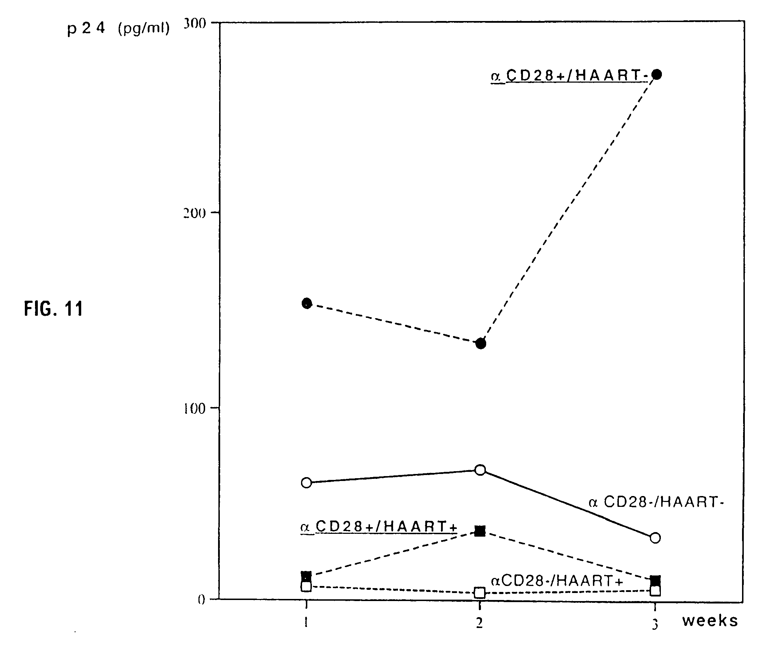

Untersucht wurde die Virusreplikation in PBMC von HIV-1 infizierten Personen. In Wochenabständen wurde freier Überstand mittels ELISA auf die Gegenwart von p24Gag (Virusprotein) untersucht. HAART wurde wie in Beispiel 2.5 verwendet. "+" bedeutet hier und folgend Einsatz der betreffenden Wirkstoffkomponente, "-" Abwesenheit. Man erkennt in der Fig. 11, daß die aCD28 Stimulation auch eine massive Virusproduktion induziert. Bei gleichzeitiger HAART ist die Virusproduktion jedoch vollständig unterdrückt. PHA ist Phytohämagglutinin zum Vergleich.

### 2.7: Verlust der HIV-1 Produktion nach Behandlung mit aCD28 und HAART.

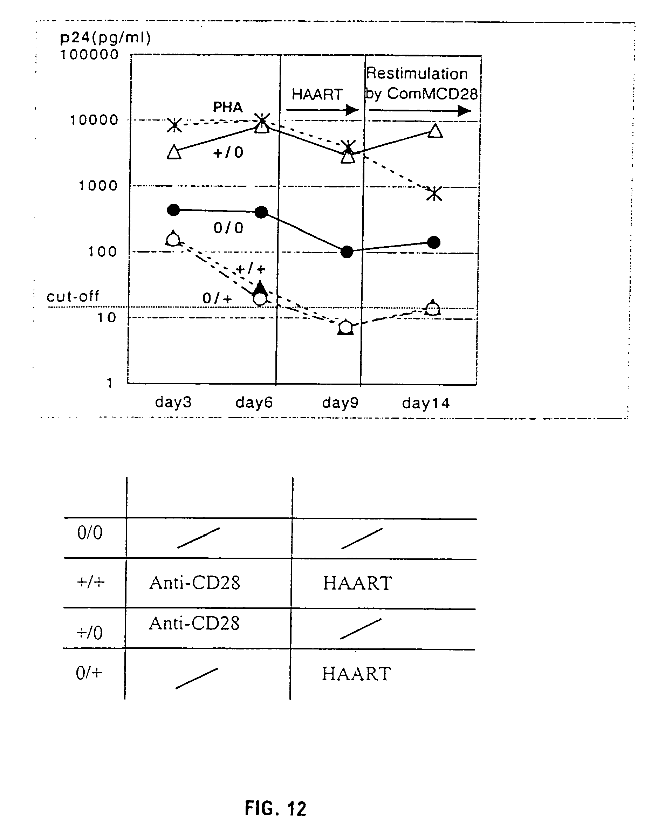

In der Figur 12 sind Ergebisse einer Versuchsreihe dargestellt, wobei PBMC von einer HIV-1 infizierten Person bis zum Tag 6 gemäß der Tabelle behandelt wurden. Vom Tag 6 bis zum Tag 9 erfolgte eine reine HAART Behandlung, gefolgt von einer Behandlung mit aCD28 (auch ComMCD28 genannt) vom Tag 9 bis zum Tag 14. Die Ergebnisse bestätigen jene aus Beispiel 2.7, da demgemäß auch nach erneuter Stimulation von Zellkulturen HIV-infizierter PBMC nach Beendigung der HAART Therapie keine Virusproduktion beobachtet wird.

### 2.8: Verlust auch proviraler HIV DNA nach Behandlung mit aCD28 und HAART.

PBMC welche von einer HIV-1 infizierten Person isoliert wurden, wurden entsprechend der Tabelle kultiviert und in vitro behandelt (Wirkstoffe und Wirkstoffmengen wie in Beispiel 2.5). Am 11. Tag der Kultivierung wurde eine HIV-1 spezifische DNA-PCR durchgeführt:
a) extern; primer:
   JA4(gag1319-1338): gaa ggc ttt cag ccc aga ag
   JA7 (gag1615-1596): tct cct act ggg ata ggt gg
   Annealing Temp.: 47°C; 42 Zyklen
b) nested; primer:
   JA5 (gag1446-1465): acc atc aat gag gaa gct gc
   JA6 (gag1577-1558): tat ttg ttc ctg aag ggt ac
   Annealing Temp.: 45°C; 25 Zyklen.

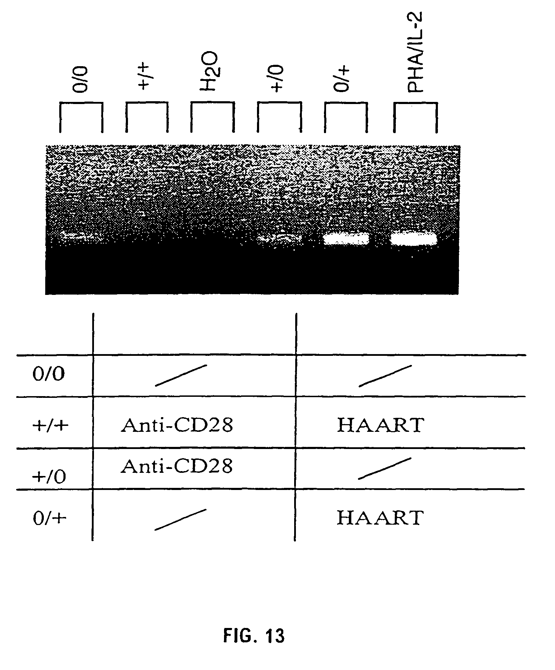

In der Figur 13 hierzu erkennt man, daß provirale HIV DNA bei kombinierter Behandlung mit HAART und erfindungsgemäß eingesetzten monoklonalen Antikörpern nicht mehr nachweisbar ist, während in den anderen Fällen der Tabelle stets provirale HIV DNA präsent war. Dies demonstriert in einem der Situation des Gesamtorganismus sehr nahe kommenden Testsystem die erfolgreiche Zerstörung auch des Reservoirs an proviralen Strukturen durch die Erfindung.

## Patentansprüche

1. Pharmazeutische Zusammensetzung in einer Ausführungsform als Präparat oder als Präparatepaket, mit pharmazeutisch wirksamen Dosen der folgenden Wirkstoffkomponenten:
a) einem monoklonalen Antikörper, welcher für Human-CD28 spezifisch ist und - Human-T-Lymphozyten mehrerer bis aller Untergruppen ohne Besetzung eines Antigenrezeptors der T-Lymphozyten und somit antigenunspezifisch aktiviert,
b) einem Reverse Transkriptase Inhibitor und
c) einem Protease Inhibitor,

2. Pharmazeutische Zusammensetzung nach Anspruch 1, wobei die Wirkstoffkomponenten b) und c) gemäß der HAART Therapie ausgewählt, dosiert und darreichungsfertig hergerichtet sind.

3. Pharmazeutische Zusammensetzung nach Anspruch 1 oder 2, wobei
die Wirkstoffkomponente b) ein Pyrimidin-Nukleosidanalog, vorzugsweise 3'-Azido-3'-desoxythymidin, ist und/oder
die Wirkstoffkomponente c) 3TC ist.

4. Pharmazeutische Zusammensetzung nach einem der Ansprüche 1 bis 4, wobei die Wirkstoffkomponenten b) und c) Bestandteil einer ersten Paketkomponente und die Wirkstoffkomponente a) Bestandteil einer zweiten Paketkomponente sind.

## Claims

1. A pharmaceutical composition in an embodiment as a preparation or as a preparation packet, comprising pharmaceutically effective dosages of the following active substance components:
a) a monoclonal antibody that is specific for human CD28 and activates human T lymphocytes of several to all subgroups without occupation of an antigen receptor of the T lymphocytes and thus activates antigen-unspecifically,
b) a reverse transcriptase inhibitor, and
c) a protease inhibitor.

2. The pharmaceutical composition according to claim 1, wherein the active substance components b) and c) are selected, dosed and prepared ready for administration according to the HAART therapy.

3. The pharmaceutical composition according to claim 1 or 2,
wherein the active substance component b) is a pyrimidine nucleoside analog, preferably 3'-azido-3'-desoxythymidine, and/or
wherein the active substance component c) is 3TC.

4. The pharmaceutical composition according to one of claims 1 to 3, wherein the active substance components b) and c) are constituents of a first packet component and the active substance component a) is a constituent of a second packet component.

## Revendications

1. Composition pharmaceutique dans une forme d'exécution comme une préparation ou comme un paquet de préparations, comprenant des dosages pharmaceutiquement efficaces de composants de substances actives suivants:
a) un anticorps monoclonal, qui est spécifique pour CD28 humain, et active des lymphocytes T humains de plusieurs à tous les sous-groupes sans occupation d'un récepteur d'antigène des lymphocytes T et donc active d'une manière non spécifique pour l'antigène,
b) un inhibiteur de transcriptase inverse, et
c) un inhibiteur de protéase.

2. Composition pharmaceutique selon la revendication 1, dans laquelle les composants de substances actives b) et c) sont choisis, dosés et préparés pour l'administration selon la thérapie HAART.

3. Composition pharmaceutique selon la revendication 1 ou 2,
dans laquelle le composant de substances actives b) est un analogue de nucléosides pyrimidiques, de préférence 3'-azido-3'-désoxythymidine, et/ou
dans laquelle le composant de substances actives c) est 3TC.

4. Composition pharmaceutique selon une des revendications 1 à 3, dans laquelle les composants de substances actives b) et c) sont des constituants d'un premier composant de paquet et le composant de substances actives a) est un constituant d'un deuxième composant de paquet.
